# DEMANDE DE BREVET EUROPEEN

(11) **EP 1 104 653 A1**
(43) Date de publication de la demande: **06.06.2001**
(21) Numéro de dépôt: 00403379.1
(22) Date de dépôt: 01.12.2000
(51) Int. Cl.: A23K 1/16, A61K 31/325, A61P 1/00

(54) **Composition pour animaux contenant du salicylate de méthyle**

(30) Priorité: 03.12.1999 FR 9915274
(71) Demandeur: Elpronat, 85600 Bouffere (FR)
(72) Inventeur: Melin, Gisèle, 44400 Reze (FR)
(74) Mandataire: Dawidowicz, Armand

(57) **Abrégé**

L'invention concerne une composition administrable par voie orale aux animaux.

Cette composition est caractérisée en ce qu'elle contient comme agent actif pour empêcher le développement d'infections du tube digestif des animaux au moins du salicylate de méthyle.

## Description

La présente invention concerne de manière générale l'utilisation du salicylate de méthyle pour la fabrication d'un agent prévenant ou empêchant la prolifération de bactéries pathogènes à l'intérieur du tube digestif d'animaux et, en particulier, une composition administrable aux animaux en vue de prévenir ou empêcher le développement d'infections du tube digestif des animaux.

Les élevages d'animaux, en particulier les élevages avicoles ou de lapins, se sont transformés depuis plusieurs années en élevages industriels intensifs au détriment des conditions d'élevage. Cette évolution s'est traduite pour les animaux par une augmentation des infections qui nuisent au gain de poids et augmentent les frais vétérinaires et le taux de mortalité.

Le but de la présente invention est de proposer comme agent prévenant ou empêchant le développement d'infections, un salicylate jamais utilisé à ce jour pour ce type d'application.

Un autre but de la présente invention est de proposer une composition administrable aux animaux simple à préparer et contenant l'élément actif en quantité très faible.

A cet effet, l'invention a pour objet une composition administrable par voie orale aux animaux, caractérisée en ce qu'elle contient, comme agent actif pour empêcher le développement d'infections du tube digestif des animaux, au moins du salicylate de méthyle.

L'invention a encore pour objet l'utilisation du salicylate de méthyle pour la fabrication d'un agent actif pour prévenir ou empêcher la prolifération de bactéries pathogènes à l'intérieur du tube digestif d'animaux.

Le salicylate de méthyle permet d'obtenir à très faible dose des effets visibles sur l'état sanitaire des animaux. Du fait de l'utilisation en faible dose, il en résulte des possibilités d'intervention parfaitement adaptées à un élevage en masse.

Selon une forme de réalisation préférée de l'invention, la composition est constituée d'au moins un mélange de salicylate de méthyle et d'au moins un support nutritif pour former un prémélange alimentaire incorporable à l'alimentation des animaux.

Le mélange de salicylate de méthyle avec un support nutritif permet une absorption maximale du salicylate de méthyle sans agresser les parois du tube digestif. Par ailleurs, ce mélange permet l'utilisation de faibles doses de salicylate de méthyle de telle sorte que la composition alimentaire obtenue est non toxique.

L'invention sera bien comprise à la lecture de la description suivante d'exemples de réalisation.

Les propriétés anti-rhumatismales et analgésiques du salicylate de méthyle sont connues depuis de nombreuses années. En effet, la gaulthérie du Canada, encore appelée gaulthéria procumbens est utilisée depuis longtemps à des fins médicinales. L'huile essentielle de cette plante est composée à 90 % de salicylate de méthyle. C'est donc à cette huile que la gaulthérie doit ses propriétés anti-inflammatoire et légèrement analgésiques. Cette huile, encore appelée essence de wintergreen, a longtemps été utilisée sous forme d'onguents, de baumes pour soulager temporairement les douleurs arthritiques et musculaires ainsi que la sciatique. Depuis plusieurs années, il est également possible de produire de l'essence artificielle de wintergreen qui contient 100 % de salicylate de méthyle. Le salicylate de méthyle est donc connu à ce jour uniquement pour ses propriétés analgésiques et anti-rhumatismales et à des fins secondaires pour ses propriétés de diurétique.

Les inventeurs de la présente invention ont découvert que, de manière surprenante, le salicylate de méthyle sous son appellation essence de wintergreen naturelle ou artificielle pouvait être utilisé pour empêcher ou limiter le développement d'infections au niveau du tube digestif des animaux. Cette utilisation du salicylate de méthyle dans une composition administrable par voie orale va à l'encontre d'un grand nombre de préjugés concernant le salicylate de méthyle pour lequel la voie orale n'a jamais été explorée, ce produit étant connu pour être mal supporté par le tube digestif.

Les inventeurs ont donc découvert que, de manière surprenante, en associant le salicylate de méthyle à au moins un support nutritif, on obtenait une prévention ou on empêchait le développement d'infections d'origine bactérienne dans le tube digestif des animaux. Il est à noter que le salicylate de méthyle est toujours utilisé dans ce qui suit ou ce qui précède sous sa forme commerciale essence de wintergreen naturelle ou artificielle.

Les inventeurs ont donc découvert que le salicylate de méthyle possède des propriétés antiseptiques et bactériostatiques qui empêchent, dans le milieu intestinal, la prolifération de bactéries pathogènes qui ont tendance à se multiplier rapidement en situation de stress des animaux. On constate ainsi que la composition, administrée aux animaux, permet, dans le cas d'animaux sains, de renforcer leur métabolisme et leur résistance immunitaire en vue d'obtenir un meilleur rendement de la production et, dans le cas d'une administration à des animaux malades, permet une amélioration rapide des éléments affectés de troubles digestifs et d'infections, notamment pour reconstitution rapide d'une flore saine du tube digestif et des intestins.

Bien que l'action du salicylate de méthyle ne soit pas encore parfaitement connue, les inventeurs pensent que le salicylate de méthyle, en empêchant ou en limitant la prolifération des micro-organismes pathogènes en particulier les coliformes dans le tube digestif, permet une colonisation de ce tube digestif par les lactobacilles. Le salicylate de méthyle agit donc sur le type de micro-organismes colonisant le tube digestif.

Cette composition administrable par voie orale aux animaux à base de salicylate de méthyle peut revêtir un grand nombre de formes de réalisation. Ainsi, cette composition administrable par voie orale est constituée d'au moins un mélange de salicylate de méthyle et d'au moins un support nutritif pour former un prémélange alimentaire incorporable au reste de l'alimentation des animaux. Généralement, le support nutritif contient au moins de la gélatine d'origine végétale ou animale. Il contient en outre au moins des glucides et éventuellement des huiles végétales. Un exemple de composition d'un support nutritif est fourni ci-après :

| | |
|---|---|
| gélatine alimentaire végétale ou animale | 20 - 45 % en poids |
| eau | 20 - 45 % en poids |
| glucose | 5 - 25 % en poids |
| huile végétale | 5 - 25 % en poids |

A titre d'huile végétale, on peut citer par exemple l'huile d'olive.

Le support représente généralement 60 à 90 % de la composition finale. Le salicylate de méthyle est ajouté dans des proportions variant de 0,0001 à 40 % en poids. D'autres additifs, tels que de l'extrait de menthol et des principes actifs de plante sous forme hydroalcoolique peuvent également être ajoutés à la composition pour compléter cette dernière.

Lorsque la composition précitée est incorporée dans une alimentation solide ou pâteuse, on mélange la composition à un support alimentaire pour former un prémélange (prémix) qui sera ultérieurement mélangé à l'aliment final de telle sorte que la concentration en salicylate de méthyle par kg d'aliment final ingéré est de préférence comprise dans la plage [0,4 g - 1,1 g].

Dans le cas où la composition doit être incorporée à l'eau de boisson des animaux, cette composition est alors constituée d'au moins un mélange de salicylate de méthyle et d'un support sous forme d'une solution nutritive aqueuse pour former une solution buvable directement incorporable à l'eau de boisson des animaux à raison de 0,0001 % à 0,020 % en poids de salicylate de méthyle par litre d'eau de boisson. La solution nutritive aqueuse peut présenter une composition analogue à celle qui a été décrite ci-dessus pour le support nutritif qui se présente sous forme liquide.

Des essais ont été réalisés pour démontrer l'action du salicylate de méthyle sur des poulets. Les essais ont démontré un gain moyen quotidien en augmentation de + 2,5 %, un indice de consommation en baisse de 8 %, un pourcentage de saisis réduit de 35 % et une réduction des frais d'intrants incluant les frais vétérinaires et les frais liés à la diététique de 38 % par animal.

Dans le cas d'une composition alimentaire présentant la composition suivante exprimée en pourcentage en poids :

| | |
|---|---|
| eau | 28 % |
| gélatine végétale ou animale | 30 % |
| dextrose | 10 % |
| huile végétale et additifs | 20 % |
| salicylate de méthyle | 12 % |

Les doses et durées d'utilisation peuvent être les suivantes lorsque cette composition est introduite dans l'eau de boisson :

| POULET | | |
|---|---|---|
| Période (en jour) | Dose/1000 litres d'eau de boisson | Durée |
| Du 1^{er} au 4^{ème} | 1 kg | 4 jours |
| du 16^{ème} au 19^{ème} | 1 kg | 4 jours |
| du 31^{ème} au 32^{ème} | 1 kg | 2 jours |
| du 55^{ème} au 56^{ème} | 1 kg | 2 jours |

| DINDE | | |
|---|---|---|
| Période (en jour) | Dose/1000 litres | Durée |
| Démarrage | 1 kg | 4 jours |
| du 26^{ème} au 29^{ème} | 1 kg | 4 jours |
| du 52^{ème} au 55^{ème} | 1 kg | 4 jours |
| après le départ des femelles | 1 kg | 2 jours |

| CANARD | | |
|---|---|---|
| Période (en jour) | Dose/1000 litres | Durée |
| Démarrage | 1 kg | 4 jours |
| du 24^{ème} au 27^{ème} | 1 kg | 4 jours |
| du 42^{ème} au 43^{ème} | 1 kg | 4 jours |

| PINTADE | | |
|---|---|---|
| Période (en jour) | Dose/1000 litres | Durée |
| Démarrage | 1 kg | 4 jours |
| du 26^{ème} au 29^{ème} | 1 kg | 4 jours |
| du 52^{ème} au 55^{ème} | 1 kg | 4 jours |

Dans tous les cas mentionnés ci-dessus, la composition précitée est diluée dans l'eau de boisson. Cette composition peut être encore utilisée au démarrage en engraissement pour les porcs. Dans ce cas là, elle sera utilisée sous forme de trois cycles successifs de cinq jours s'effectuant tous les 25 jours. La dose à fournir aux animaux sera de 0,1 g de la composition précitée par kg de poids vif par jour. Cette composition alimentaire pourra également être utilisée en fin de gestation pour les truies à raison de 65 g de composition par jour, les jours avant mise bas ou en lactation à raison de 80 g par jour, huit jours après mise bas. Bien évidemment, les doses et périodes fournies ci-dessus ne sont fournies qu'à titre indicatif.

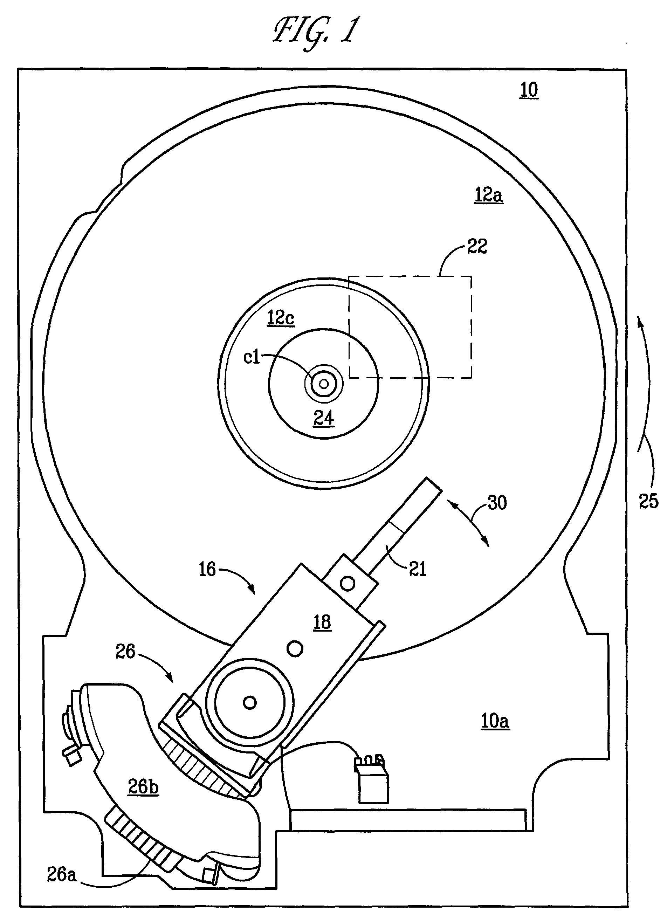

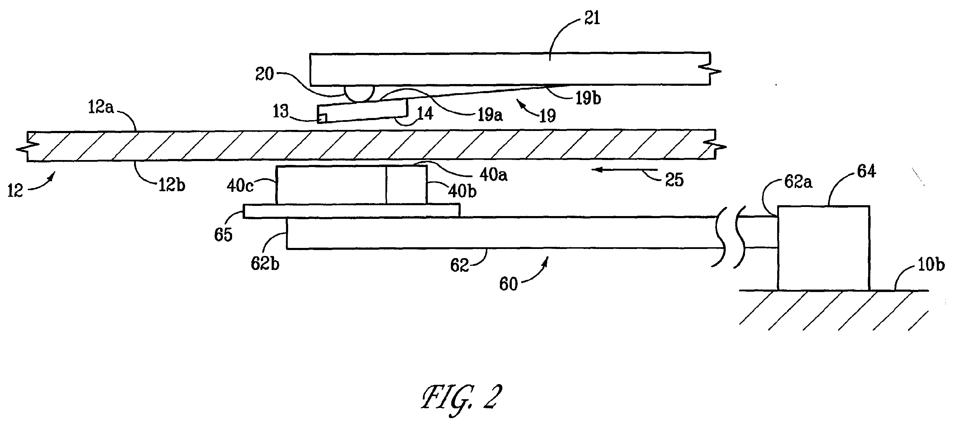

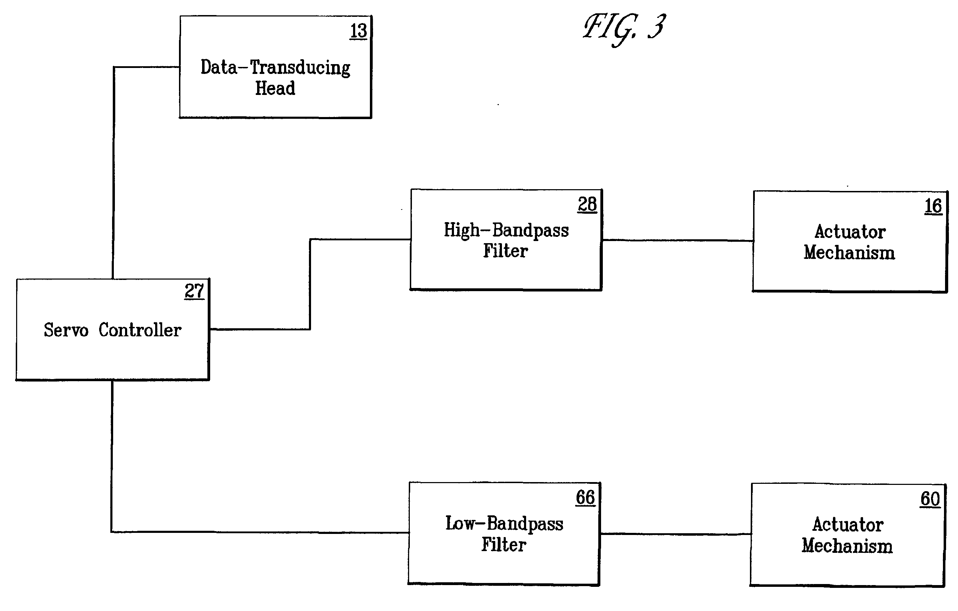

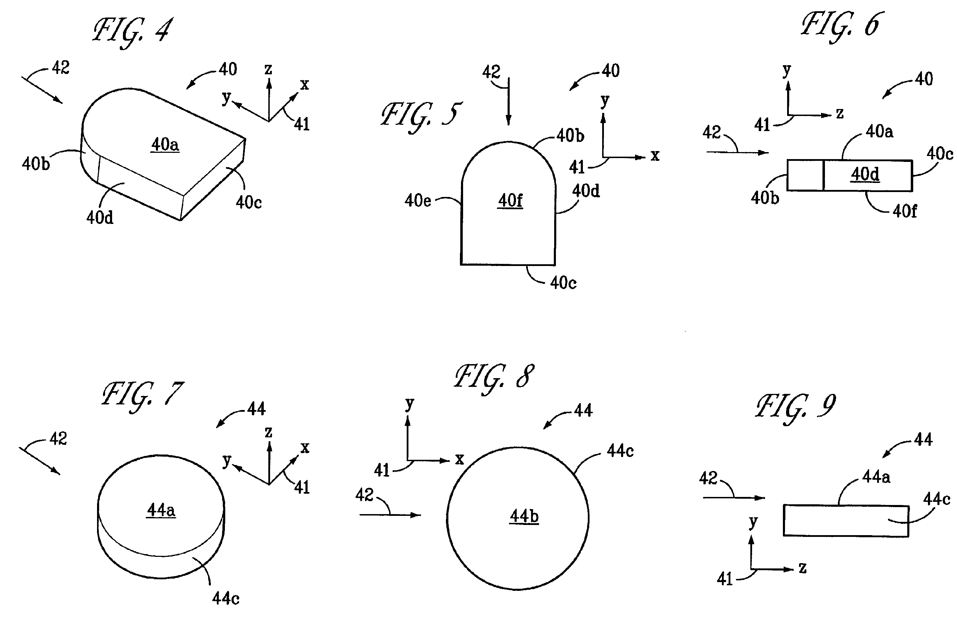

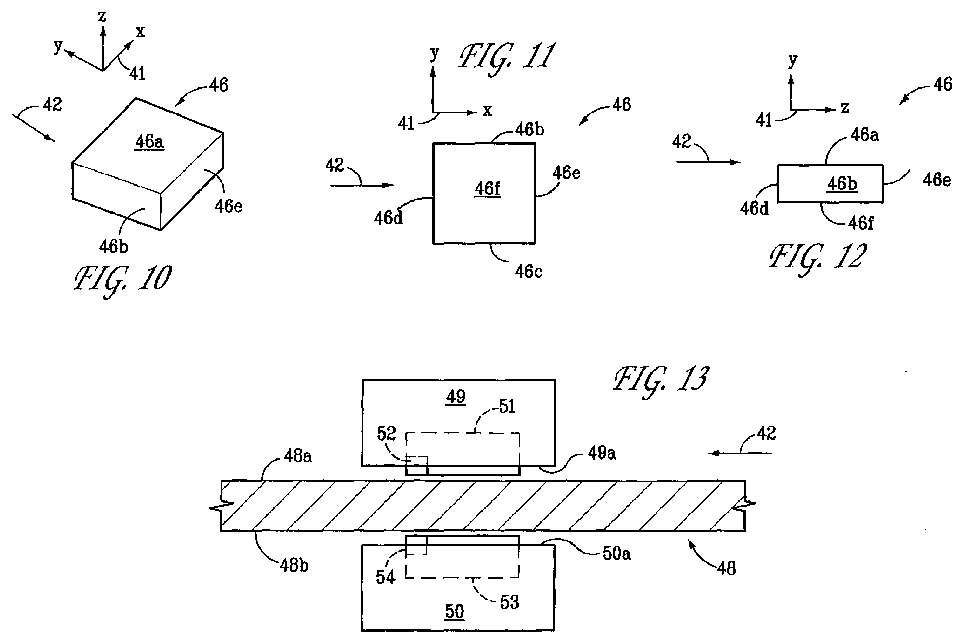

## Revendications

1. Composition administrable par voie orale aux animaux,
caractérisée en ce qu'elle contient, comme agent actif pour empêcher le développement d'infections du tube digestif des animaux, au moins du salicylate de méthyle.

2. Composition administrable par voie orale aux animaux selon la revendication 1,
caractérisée en ce qu'elle est constituée d'au moins un mélange de salicylate de méthyle et d'au moins un support nutritif pour former un prémélange alimentaire incorporable à l'alimentation des animaux.

3. Composition administrable par voie orale aux animaux selon la revendication 2,
caractérisée en ce que le support nutritif contient au moins de la gélatine d'origine végétale ou animale.

4. Composition administrable par voie orale aux animaux selon la revendication 3,
caractérisée en ce que le support nutritif contient, outre de la gélatine d'origine végétale ou animale, au moins des glucides et éventuellement des huiles végétales.

5. Composition administrable par voie orale aux animaux selon l'une des revendications 2 à 4,
caractérisée en ce que le support nutritif a pour composition :
| | |
|---|---|
| gélatine alimentaire végétale ou animale | 20 - 45 % en poids |
| eau | 20 - 45 % en poids |
| glucose | 5 - 25 % en poids |
| huile végétale | 5 - 25 % en poids |

6. Composition administrable par voie orale aux animaux selon l'une des revendications 2 à 5,
caractérisée en ce qu'elle est incorporée à un aliment final de sorte que la concentration en salicylate de méthyle par kg d'aliment final ingéré est de préférence comprise dans la plage [0,4 g - 1,1 g].

7. Composition administrable par voie orale aux animaux selon l'une des revendications 1 à 5,
caractérisée en ce qu'elle est constituée d'au moins un mélange de salicylate de méthyle et d'un support sous forme d'une solution nutritive aqueuse pour former une solution buvable directement incorporable à l'eau de boisson des animaux à raison de 0,0001 % % à 0,020 % en poids de salicylate de méthyle par litre d'eau de boisson.

8. Utilisation du salicylate de méthyle pour la fabrication d'un agent actif pour prévenir ou empêcher la prolifération de bactéries pathogènes à l'intérieur du tube digestif d'animaux.
